# EUROPEAN PATENT APPLICATION

(11) **EP 4 183 866 A1**
(43) Date of publication of application: **24.05.2023**
(21) Application number: 21842751.6
(22) Date of filing: 16.07.2021
(51) Int. Cl.: C12N 5/00, A61K 35/32, A61K 35/35, A61K 35/51, A61K 38/43, A61P 31/14, A61P 43/00

(54) **COMPOSITION, BINDING INHIBITOR, MEDICAL DEVICE, AND METHOD FOR PREVENTING COVID-19**

(30) Priority: 17.07.2020 WO PCT/JP2020/027874; 18.11.2020 JP 2020191759
(71) Applicant: Dexon Pharmaceuticals Inc., Tokyo, 103-0027 (JP)
(72) Inventor: KOGA Shoji, Tokyo 103-0027 (JP)
(74) Representative: Godemeyer Blum Lenze Patentanwälte Partnerschaft mbB - werkpatent
(86) International application number: PCT/JP2021/026716
(87) International publication number: WO 2022/014693

(57) **Abstract**

A composition containing a culture supernatant of dental pulp-derived stem cells, adipose-derived stem cells, umbilical cord-derived stem cells or immortalized stem cells thereof in which the composition contains angiotensin-converting enzyme 2 (ACE2) and is used for administration to a healthy person or a healthy animal for preventing infection of the healthy person or the healthy animal with a virus that uses ACE2 as a receptor can be used as a medicine for preventing infection with a virus that uses ACE2 as a receptor, such as COVID-19, or the like when administered to a healthy person or a healthy animal.

## Description

### Technical Field

The present invention relates to a composition, a binding inhibitor, a medical device and a prevention method of COVID-19.

### Background Art

SARS-CoV-2 (2019-nCoV), which is also called a new coronavirus, causes the onset of a new coronaviral infection (COVID-19). Therapeutic methods and prevention methods of COVID-19 have been studied. Of the methods, a method using mesenchymal stem cells (MSCs) has been found to be effective as a method for treating COVID-19 pneumonia (see NPLs 1 to 3).

NPL 1 discloses that mesenchymal stem cells-based immunomodulation treatment has been proposed as a suitable therapeutic approach for COVID-19. In particular, NPL 1 discloses that, after the intravenous transplantation of MSCs, a significant population of cells accumulates in the lung, which they alongside immunomodulatory effect could protect alveolar epithelial cells, reclaim the pulmonary microenvironment, prevent pulmonary fibrosis and cure lung dysfunction. On page 431 of NPL 1, it is stated that ACE2-negative MSCs are preferably used in the document [25] cited in NPL 1.

NPL 2 discloses that umbilical cord mesenchymal stem cells, through the antiinflammatory and immunomodulatory actions, can heal tissues thereby enhancing recovery from COVID-19. NPL 2 further states that development of medicines that bind to ACE2 is required because SARS-CoV-2, like SARS-CoV, binds to cells having angiotensin-converting enzyme 2 (ACE2) (alveolar type II cells, capillary endothelial cells and the like) (also see J. Biol. Chem., 2005 Aug 26, 280(34), pp.30113-30119).

NPL 3 discloses that the intravenous transplantation of ACE2⁻ (ACE2-free) mesenchymal stem cells was safe and effective for treatment in patients with COVID-19 pneumonia, especially for patients in critically severe condition.

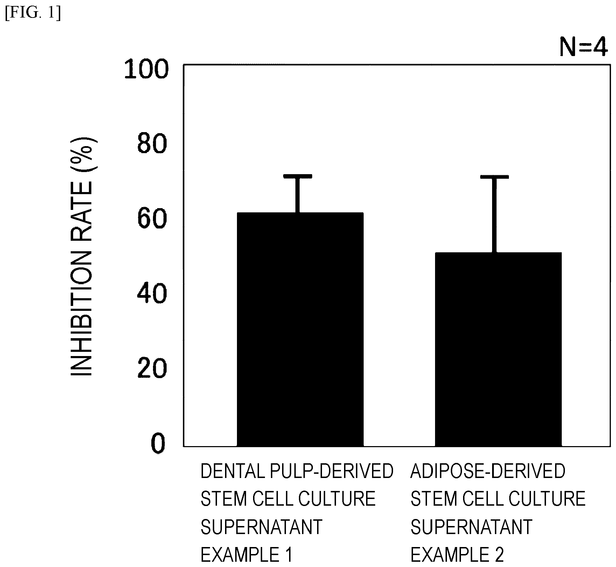

NPL 4 describes a clinical trial plan for treating COVID-19 using MSCs but does not clearly disclose whether it was effective as a therapeutic medicine. The MSCs in FIGURE 1 of NPL 4 are ACE2 negative, and the document [26] which describes that ACE2-negative bone marrow-derived mesenchymal stem cells (MSCs) are preferably used is cited at line 4 in the left column on page 7 (the document [26] of NPL 4 is the same as the document [25] of NPL 1). Moreover, NPL 4 states that the expression of ACE2 in other sources is unclear.

NPL 5 also describes a clinical trial plan for treating COVID-19 using MSCs but does not clearly disclose whether it was effective as a therapeutic medicine. In this regard, NPL 5 does not describe ACE2.

### Citation List

### Non Patent Literature

NPL 1: Stem Cell Rev Rep., 2020 Apr 13, Vol. 16, pp.427-433
NPL 2: Pain Physician, 2020 Mar 23, 23(2), E71-E83
NPL 3: Aging and Disease, 2020 Apr, 11(2), pp.216-228
NPL 4: Frontiers in Immunology, 2020 July 03, Vol.11, 1563, pp.1-10
NPL 5: Expert Opinion on Biological Therapy, 2020 April 29, 20(7), pp.711-716

### Summary of Invention

### Technical Problem

NPLs 1 to 5, however, could not sufficiently identify the active ingredient of mesenchymal stem cells or the therapeutic mechanism for COVID-19. Moreover, NPLs 1 to 5 have focused on use of mesenchymal stem cells in the clinical stage, namely use in a method for treating patients hospitalized with any symptoms of COVID-19, and these documents have not focused on the administration to healthy individuals at all. Accordingly, NPLs 1 to 5 do not elucidate any method for preventing COVID-19 using mesenchymal stem cells.

A problem that the invention is to solve is to provide a composition which can be used as a medicine for preventing infection with a virus that uses ACE2 as a receptor, such as COVID-19, or the like when administered to a healthy person or a healthy animal.

### Solution to Problem

As a result of intensive studies to solve the problem, the inventor and the like have experimentally confirmed that binding between a virus that uses ACE2 as a receptor and ACE2 can be inhibited using a culture supernatant of specific mesenchymal stem cells and that the problem can be thus solved. The inventor and the like have found that particularly preferably exosomes derived from a culture supernatant of specific mesenchymal stem cells express and contain ACE2 and that the problem can be solved by inhibiting or blocking the attachment of SARS-CoV-2 to living cells using the exosomes.

It is disclosed in NPLs 1 and 4 that ACE2-negative MSCs are preferably used in the treatment of COVID-19, but the matter that COVID-19 in healthy individuals could be prevented rather using an ACE2-positive composition, exosomes and the like cannot be read from NPLs 1 and 4. Even referring to documents which describe a clinical trial plan for treating viral infection, the efficacy as a medicine for preventing the viral infection of healthy individuals cannot be read without any experimental confirmation.

The invention and preferable constitution of the invention are specifically as follows.
[1] A composition containing a culture supernatant of dental pulp-derived stem cells, adipose-derived stem cells, umbilical cord-derived stem cells or immortalized stem cells thereof,
   wherein the composition contains angiotensin-converting enzyme 2 (ACE2) and
   is used for administration to a healthy person or a healthy animal.
[1-1] A composition containing a culture supernatant of dental pulp-derived stem cells, adipose-derived stem cells, umbilical cord-derived stem cells or immortalized stem cells thereof,
   wherein the composition contains angiotensin-converting enzyme 2 (ACE2) and
   is used for administration to a healthy person or a healthy animal for preventing infection of the healthy person or the healthy animal with a virus that uses ACE2 as a receptor.
[2] A composition containing microparticles derived from a culture supernatant of dental pulp-derived stem cells, adipose-derived stem cells, umbilical cord-derived stem cells or immortalized stem cells thereof,
   wherein the microparticles contain ACE2, and
   the composition is used for administration to a healthy person or a healthy animal.
[2-1] A composition containing microparticles derived from a culture supernatant of dental pulp-derived stem cells, adipose-derived stem cells, umbilical cord-derived stem cells or immortalized stem cells thereof,
   wherein the microparticles contain ACE2, and
   the composition is used for administration to a healthy person or a healthy animal for preventing infection of the healthy person or the healthy animal with a virus that uses ACE2 as a receptor.
[3] The composition described in [2] or [2-1], wherein the microparticles are microparticles derived from the culture supernatant of dental pulp-derived stem cells.
[4] The composition described in any one of [2] to [3] (including [2-1], the same applies below), wherein the microparticles are exosomes.
[5] The composition described in any one of [2] to [4], wherein the microparticles are microparticles purified from the culture supernatant.
[6] The composition described in any one of [2] to [5] which contains 0.01×10⁸ particles/ml or more of the microparticles.
[7] The composition described in any one of [1] to [6] (including [1-1] and [2-1], the same applies below) which contains 100 ng/ml or more of ACE2.
[8] The composition described in any one of [1] to [7], wherein the dental pulp-derived stem cells, the adipose-derived stem cells or the umbilical cord-derived stem cells used for the culture supernatant are not genetically modified.
[9] The composition described in any one of [1] to [8] which is used for inhibiting binding of a virus that uses ACE2 as a receptor to ACE2 of a human or a nonhuman animal.
[10] The composition described in any one of [1] to [9], wherein the inhibition rate of binding between the spike protein of a virus that uses ACE2 as a receptor and ACE2 of a human or a nonhuman animal is 40% or more.
[11] The composition described in [9] or [10] which is used for inhibiting cell entry or cell fusion of SARS-CoV-2 into a cell having ACE2.
[12] The composition described in any one of [1] to [11] which is a medicine for preventing COVID-19 in the healthy person or the healthy animal.
[13] The composition described in any one of [1] to [12], wherein the dental pulp-derived stem cells, the adipose-derived stem cells or the umbilical cord-derived stem cells are human dental pulp-derived stem cells, human adipose-derived stem cells or human umbilical cord-derived stem cells.
[14] A binding inhibitor containing a culture supernatant of dental pulp-derived stem cells, adipose-derived stem cells, umbilical cord-derived stem cells or immortalized stem cells thereof,
   wherein the binding inhibitor contains angiotensin-converting enzyme 2 (ACE2),
   is used for administration to a healthy person or a healthy animal and
   inhibits binding of a virus that uses ACE2 as a receptor to ACE2 of a human or a nonhuman animal.
[15] A binding inhibitor containing microparticles derived from a culture supernatant of dental pulp-derived stem cells, adipose-derived stem cells, umbilical cord-derived stem cells or immortalized stem cells thereof,
   wherein the microparticles contain ACE2, and
   the binding inhibitor is used for administration to a healthy person or a healthy animal and
   inhibits binding of a virus that uses ACE2 as a receptor to ACE2 of a human or a nonhuman animal.
[16] A medical device containing at least one of the composition described in any one of [1] to [13] and the binding inhibitor described in [14] or [15].
[17] A method for preventing COVID-19 including a step of administering at least one of the composition described in any one of [1] to [13] and the binding inhibitor described in [14] or [15] to a human or a nonhuman animal or a step of installing the medical device described in [16] in a human or a nonhuman animal.

### Advantageous Effects of Invention

According to the invention, a composition which can be used as a medicine for preventing infection with a virus that uses ACE2 as a receptor, such as COVID-19, or the like when administered to a healthy person or a healthy animal can be provided.

### Brief Description of Drawings

[Fig. 1] Fig. 1 is a graph showing the inhibition rates of spike-ACE2 binding of the compositions of Examples (culture supernatants).
[Fig. 2] Fig. 2 is a graph showing the inhibition rate of spike-ACE2 binding of the composition of Example 11 (exosomes).
[Fig. 3] Fig. 3 is a graph showing the relative expression levels of ACE2 in the exosomes contained in the compositions of Examples and a Reference Example where the expression level in PNT2 EVs used as a positive control is regarded as 100.

### Description of Embodiments

The invention is explained in detail below. The constituent features described below are sometimes explained based on typical embodiments or specific examples, but the invention should not be limited to the embodiments. In the present specification, a numerical range expressed using "to" means a range including the values before and after "to" as the lower limit and the upper limit.

### [Composition]

In a first aspect, the composition of the invention is a composition containing a culture supernatant of dental pulp-derived stem cells, adipose-derived stem cells, umbilical cord-derived stem cells or immortalized stem cells thereof (also called dental pulp-derived stem cells or the like below), and the composition contains angiotensin-converting enzyme 2 (ACE2) and is used for administration to a healthy person or a healthy animal.

In a second aspect, the composition of the invention is a composition containing microparticles derived from a culture supernatant of dental pulp-derived stem cells, adipose-derived stem cells, umbilical cord-derived stem cells or immortalized stem cells thereof (also called dental pulp-derived stem cells or the like below). The microparticles contain angiotensin-converting enzyme 2 (ACE2), and the composition is used for administration to a healthy person or a healthy animal.

The composition of the invention is preferably used for administration to a healthy person or a healthy animal for preventing infection of the healthy person or the healthy animal with a virus that uses ACE2 as a receptor (such as COVID-19).

The composition of the invention can be used as a medicine for preventing infection with a virus that uses ACE2 as a receptor, such as COVID-19, or the like when administered to a healthy person or a healthy animal.

Preferable aspects of the composition of the invention are explained below.

### <Microparticles>

In the invention, microparticles derived from a culture supernatant of dental pulp-derived stem cells or the like are used. The microparticles are derived from dental pulp-derived stem cells or the like, for example, through secretion, emergence, dispersion or the like from the dental pulp-derived stem cells or the like and are exuded or released or fall into a cell culture medium. Thus, the microparticles are contained in a culture supernatant of dental pulp-derived stem cells or the like.

When the composition of the invention is used, the microparticles derived from a culture supernatant of dental pulp-derived stem cells, adipose-derived stem cells or immortalized stem cells thereof may be contained in the culture supernatant or may be purified from the culture supernatant. That is, the culture supernatant of dental pulp-derived stem cells or the like may be used as the composition of the invention, or a composition containing microparticles purified from the culture supernatant may be used as the composition of the invention.

In the invention, rather than using microparticles purified from a culture supernatant as the composition of the invention, a culture supernatant of dental pulp-derived stem cells or the like is preferably used as the composition of the invention because the inhibition rate of spike-ACE2 binding is high.

In the first aspect of the composition of the invention, the composition preferably contains angiotensin-converting enzyme 2 (ACE2), and the microparticles preferably contain ACE2. In the second aspect of the composition of the invention, the microparticles express and contain ACE2. ACE2 is a zinc metalloprotease of the ACE family and is an important regulatory factor of the renin-angiotensin system. The tissue distribution of ACE2 is more restricted than that of ACE, and ACE2 is mainly found in heart, kidney, testis, epithelial cells (mucosal cells) in respiratory tract, lung and intestinal tract, alveolar type II cells, capillary endothelial cells and the like.

Because the main component which contains ACE2 is supposed to be microparticles, it is generally expected that purified microparticles would exhibit a higher inhibition rate of spike-ACE2 binding than a culture supernatant of dental pulp-derived stem cells or the like when the total protein amounts are the same. In the invention, however, it was surprisingly found that culture supernatants of dental pulp-derived stem cells or the like (especially a culture supernatant of dental pulp-derived stem cells) have a higher inhibition rate of spike-ACE2 binding than purified microparticles, and this point was actually confirmed thorough binding inhibition experiment.

### (Characteristics of Microparticles)

The microparticles are preferably of at least one kind selected from the group consisting of exosomes, microvesicles, membrane particles, membrane vesicles, ectosomes, exovesicles and microvesicles, more preferably exosomes.

The diameter of the microparticles is preferably 10 to 1000 nm, more preferably 30 to 500 nm, particularly preferably 50 to 150 nm.

The microparticles preferably express and have ACE2 on the surface because binding to a virus that uses ACE2 as a receptor is easier.

Moreover, molecules called tetraspanins such as CD9, CD63 and CD81 are desirably on the surface of the microparticles, and the molecules may be those of CD9 alone, CD63 alone, CD81 alone or any combination of two or three thereof.

A preferable aspect in which exosomes are used as the microparticles is sometimes explained below, but the microparticles of the invention are not limited to exosomes.

The exosomes are preferably extracellular vesicles which are released from cells during fusion of plasma membrane and multivesicular bodies.

The surface of the exosomes preferably contains a lipid and a protein derived from the cell membrane of dental pulp-derived stem cells or the like.

The exosomes preferably contain, in their inside, an intracellular substance of dental pulp-derived stem cells or the like such as nucleic acids (microRNAs, messenger RNAs, DNAs and the like) and proteins.

The exosomes are known to be used for communication among cells through transportation of genetic information from a cell to another cell. The exosomes can be tracked easily and can be targeted at a specific region.

### (Microparticle Content)

The microparticle content of the composition of the invention is not particularly restricted. To increase the inhibition rate of spike-ACE2 binding, the composition of the invention preferably contains 0.5×10⁸ particles or more, more preferably 1.0×10⁸ particles or more, particularly preferably 2.0×10⁸ particles or more of the microparticles.

The microparticle content of the composition of the invention as the total protein amount is not particularly restricted. To increase the inhibition rate of spike-ACE2 binding, the composition of the invention preferably contains 0.5 ng or more of the microparticles as the total protein amount, more preferably 1 ng or more, particularly preferably 2 ng or more. Furthermore, when the composition of the invention contains more than 2 ng of the microparticles as the total protein amount, the inhibition rate of spike-ACE2 binding can be further increased.

The concentration of the microparticles contained in the composition of the invention is not particularly restricted. To increase the inhibition rate of spike-ACE2 binding, the composition of the invention preferably contains the microparticles at 0.01×10⁸ particles/mL or more, more preferably at 0.05×10⁸ particles/mL or more, particularly preferably 0.1×10⁸ particles/mL or more.

It is difficult to prepare a composition having a high microparticle content or a high concentration thereof. Using a culture supernatant of dental pulp-derived stem cells or the like produced by the method described below or using microparticles purified from the culture supernatant for the composition of the invention, the microparticle content or the concentration thereof is increased.

### (Culture Supernatant of Dental Pulp-Derived Stem Cells or the Like)

The culture supernatant of dental pulp-derived stem cells or the like is not particularly restricted.

The culture supernatant of dental pulp-derived stem cells or the like preferably does not substantially contain serum. For example, the serum content of the culture supernatant of dental pulp-derived stem cells or the like is preferably 1 mass% or less, more preferably 0.1 mass% or less, particularly preferably 0.01 mass% or less.

### - Dental Pulp-Derived Stem Cells or the Like -

The dental pulp-derived stem cells, the adipose-derived stem cells or the umbilical cord-derived stem cells may be derived from a human or from a nonhuman animal. The nonhuman animal may be the same as the animal (species) as the subject of the administration of the composition of the invention described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used. In addition to human deciduous dental pulp stem cells and human permanent dental pulp stem cells, dental pulp-derived stem cells derived from a nonhuman animal such as pig deciduous dental pulp stem cells can be used.

The dental pulp-derived stem cells (the same applies to the adipose-derived stem cells and the umbilical cord-derived stem cells described below) can produce, in addition to exosomes, vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-a, KGF, HBEGF, SPARC, other growth factors and various cytokines such as chemokine. Many other bioactive substances can also be produced.

In the invention, the dental pulp-derived stem cells used for the culture supernatant of dental pulp-derived stem cells particularly preferably contain many proteins, and deciduous dental pulp stem cells are preferably used. That is, in the invention, a culture supernatant of deciduous dental pulp stem cells is preferably used.

The adipose-derived stem cells used for the culture supernatant are not particularly restricted. As the adipose-derived stem cells, somatic stem cells contained in any adipose tissue can be used. In addition to human adipose-derived stem cells, adipose-derived stem cells derived from a nonhuman animal such as pig adipose stem cells can be used. As the adipose-derived stem cells, for example, adipose-derived stem cells prepared by the method described in [0023] to [0041] of WO2018/038180 can be used, and the contents of the publication are incorporated in the present specification by reference.

The umbilical cord-derived stem cells used for the culture supernatant are not particularly restricted. In addition to human umbilical cord-derived stem cells, umbilical cord-derived stem cells derived from a nonhuman animal such as pig umbilical cord stem cells can be used. As the umbilical cord-derived stem cells, for example, umbilical cord-derived stem cells prepared by the method described in [0023] to [0033] of JP-A-2017-119646 can be used, and the contents of the publication are incorporated in the present specification by reference.

The dental pulp-derived stem cells or the like used in the invention may be natural cells or genetically modified cells as long as the intended treatment can be achieved.

In particular, in the invention, immortalized stem cells of dental pulp-derived stem cells, adipose-derived stem cells or umbilical cord-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells, adipose-derived stem cells or umbilical cord-derived stem cells are not particularly restricted. The immortalized stem cells are preferably non-tumor immortalized stem cells. The immortalized stem cells of dental pulp-derived stem cells, adipose-derived stem cells or umbilical cord-derived stem cells can be prepared by adding one of the following small molecule compounds (inhibitors) or any combination thereof to dental pulp-derived stem cells, adipose-derived stem cells or umbilical cord-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidozol-4-yl)-6-methylpyridine, 3-(6-methylpyridin-2-yl)-4-(4-quinolyl)-1-phenylthiocarbamoyl-1H-pyrazole (A-83-01), 2-(5-chloro-2-fluorophenyl)pteridin-4-yl)pyridin-4-ylamine (SD-208), 3-(pyridin-2-yl)-4-(4-quinonyl)]-1H-pyrazole and 2-(3-(6-methylpyridin-2-yl)-1H-pyrazol-4-yl)-1,5-naphthyridine (all are from Merck), SB431542 (Sigma-Aldrich) and the like. A-83-01 is preferable.

ROCK inhibitors are not particularly limited as long as the ROCK inhibitors have the action of inhibiting the function of Rho-binding kinase. Examples of the ROCK inhibitors include GSK269962A (Axonmedchem), Fasudil hydrochloride (Tocris Bioscience), Y-27632 and H-1152 (both are from FUJIFILM Wako Pure Chemical Corporation) and the like. Y-27632 is preferable.

GSK3 inhibitors are not particularly limited as long as GSK-3 (glycogen synthase kinase 3) is inhibited, and examples thereof include A 1070722, BIO and BIO-acetoxime (all are from TOCRIS) and the like.

MEK inhibitors are not particularly limited as long as the MEK inhibitors have the action of inhibiting the function of MEK (MAP kinase-ERK kinase), and examples thereof include AZD6244, CI-1040 (PD184352), PD0325901, RDEA119 (BAY86-9766), SL327 and U0126-EtOH (all are from Selleck), PD98059, U0124 and U0125 (all are from Cosmo Bio Co., Ltd.) and the like.

When the composition of the invention is used for regenerative medicine, due to the requirements of the Act on the Safety of Regenerative Medicine, the composition containing microparticles derived from a culture supernatant of dental pulp-derived stem cells, adipose-derived stem cells, umbilical cord-derived stem cells or immortalized stem cells thereof (dental pulp-derived stem cells or the like) does not contain other somatic stem cells than the dental pulp-derived stem cells or the like. The composition of the invention may contain mesenchymal stem cells other than the dental pulp-derived stem cells or the like or other somatic stem cells but preferably does not contain such cells.

The mesenchymal stem cells other than the dental pulp-derived stem cells or the like include bone marrow-derived stem cells and the like but are not limited thereto.

Examples of the somatic stem cells other than the mesenchymal stem cells include stem cells derived from the dermal system, the digestive system, the myeloid system, the nerve system and the like, but the somatic stem cells are not limited to the examples. Examples of the somatic stem cells of the dermal system include epithelial stem cells, hair follicle stem cells and the like. Examples of the somatic stem cells of the digestive system include pancreatic (general) stem cells, hepatic stem cells and the like. Examples of the somatic stem cells of the myeloid system (except for the mesenchymal stem cells) include hematopoietic stem cells and the like. Examples of the somatic stem cells of the nerve system include neural stem cells, retinal stem cells and the like.

The composition of the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

### - Preparation Method of Culture Supernatant of Dental Pulp-Derived Stem Cells or the Like -

The method for preparing the culture supernatant of dental pulp-derived stem cells, adipose-derived stem cells, umbilical cord-derived stem cells or immortalized stem cells thereof is not particularly restricted, and a conventional method can be used.

The culture supernatant of dental pulp-derived stem cells or the like is a culture solution obtained by culturing dental pulp-derived stem cells or the like and does not contain the cells themselves. For example, a culture supernatant which can be used for the invention can be obtained by culturing dental pulp-derived stem cells or the like and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used.

The dental pulp-derived stem cells or the like for obtaining the culture supernatant of dental pulp-derived stem cells or the like can be screened by a normal method and can be screened based on the size and the morphology of the cells or as adherent cells. The dental pulp-derived stem cells can be screened from dental pulp cells collected from an exfoliated deciduous tooth or a permanent tooth as adherent cells or passaged cells thereof. The adipose-derived stem cells can be screened from adipose cells collected from adipose tissue as adherent cells or passaged cells thereof. The umbilical cord-derived stem cells can be screened from cells collected from an umbilical cord as adherent cells or passaged cells thereof. The culture supernatant of dental pulp-derived stem cells or the like used can be a culture supernatant obtained by culturing screened stem cells.

The "culture supernatant of dental pulp-derived stem cells or the like" is preferably a culture solution which does not contain the cells themselves obtained by culturing the dental pulp-derived stem cells or the like. The culture supernatant of dental pulp-derived stem cells or the like used in the invention preferably does not contain any cells (regardless of the kinds of cells) as a whole in an aspect. Due to the feature, the composition of the aspect is clearly distinguished of course from the dental pulp-derived stem cells or the like themselves and also from various compositions containing dental pulp-derived stem cells or the like. A typical example of the aspect is a composition which does not contain dental pulp-derived stem cells or the like and which is composed only of a culture supernatant of dental pulp-derived stem cells or the like.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is particularly preferably a composition composed only of a culture supernatant of deciduous dental pulp-derived stem cells.

A basal medium, a basal medium supplemented with serum and the like or the like can be used for the culture solution of dental pulp-derived stem cells or the like for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F12 medium (SIGMA, GIBCO and the like), RPMI1640 medium and the like. Examples of the component that can be added to the medium include serum (fetal bovine serum, human serum, sheep serum and the like), serum replacements (knockout serum replacement (KSR) and the like), bovine serum albumin (BSA), antibiotics, vitamins and minerals.

In order to prepare "a culture supernatant of dental pulp-derived stem cells or the like" which does not contain serum, however, a serum-free medium should be used throughout the whole process or for the last passaging culture or the last passaging culture steps. For example, when dental pulp-derived stem cells or the like are cultured in a medium which does not contain serum (a serum-free medium), a culture supernatant of dental pulp-derived stem cells or the like which does not contain serum can be prepared. When passaging culture is conducted once or two or more times and when the last passaging culture or the last passaging culture steps are conducted in a serum-free medium, a culture supernatant of dental pulp-derived stem cells or the like which does not contain serum can also be obtained. Moreover, a culture supernatant of dental pulp-derived stem cells or the like which does not contain serum can also be obtained by removing serum from the recovered culture supernatant using dialysis, solvent substitution using a column or the like.

For culturing the dental pulp-derived stem cells or the like for obtaining the culture supernatant, generally used conditions can be applied as they are. The method for preparing a culture supernatant of the dental pulp-derived stem cells or the like can be the same as the cell culture method described below except that the isolation and screening steps of the stem cells are appropriately adjusted to the type of the stem cells. One skilled in the art can appropriately isolate and screen dental pulp-derived stem cells or the like according to the type of the dental pulp-derived stem cells or the like.

In order to produce a large amount of a specific kind of exosomes which contributes to the expression of ACE2 and/or the ACE2 concentration (activity), special conditions may be applied for culturing the dental pulp-derived stem cells or the like. The special conditions may be, for example, low-temperature conditions, low-oxygen conditions, microgravity conditions, conditions of coculturing with any stimulant or the like.

### - Other Component Contained in Culture Supernatant of Dental Pulp-Derived Stem Cells or the Like -

The culture supernatant of dental pulp-derived stem cells or the like used for preparing exosomes in the invention may contain another component in addition to the culture supernatant of dental pulp-derived stem cells or the like but preferably does not substantially contain any other component.

The culture supernatant of dental pulp-derived stem cells or the like may be stored after an additive used for preparing exosomes is added to the culture supernatant.

### (Purification of Microparticles)

Microparticles can be purified from the culture supernatant of dental pulp-derived stem cells or the like.

The purification of the microparticles is preferably separation of a fraction containing the microparticles from the culture supernatant of dental pulp-derived stem cells or the like, more preferably isolation of the microparticles.

The microparticles can be isolated by separation from non-associated components based on the characteristics of the microparticles. For example, the microparticles can be isolated based on the molecular weight, the size, the morphology, the composition or the biological activity.

In the invention, the microparticles can be purified by centrifuging the culture supernatant of dental pulp-derived stem cells or the like and collecting a specific fraction containing a large amount of the microparticles (for example, precipitates) obtained by the centrifugation. Unnecessary components (insoluble components) in a faction other than the predetermined fraction may be removed. The solvent, the dispersion medium and the unnecessary components do not have to be removed completely from the composition of the invention. An example of the centrifugation conditions is 100 to 20000 g for 1 to 30 minutes.

In the invention, the microparticles can be purified by subjecting the culture supernatant of dental pulp-derived stem cells or the like or a centrifuged product thereof to filtration. Unnecessary components can be removed through the filtration. Moreover, when a filter membrane having an appropriate pore size is used, the removal of the unnecessary components and sterilization can be conducted simultaneously. The material, the pore size and the like of the filter membrane used for the filtration are not particularly limited. The filtration can be achieved by a known method using a filter membrane having an appropriate molecular weight or an appropriate size cut-off. Because exosomes can be easily fractionated, the pore size of the filter membrane is preferably 10 to 1000 nm, more preferably 30 to 500 nm, particularly preferably 50 to 150 nm.

In the invention, the culture supernatant of dental pulp-derived stem cells or the like, a centrifuged product thereof or a filtered product thereof can be separated further using separation means such as RAM chromatography. For example, high-performance liquid chromatography (HPLC) using any of various columns can be used. The column which can be used is a size exclusion column or a binding column.

In order to track the microparticles (or the activity thereof) in the fraction at each treatment stage, one or more characteristics or the biological activity of the microparticles can be used. For example, in order to track the microparticles, light scattering, refractive index, dynamic light scattering or UV-VIS detector can be used. Alternatively, in order to track the activity in each fraction, the expression level of ACE2, the activity of ACE2, the concentration of ACE2 or the like can be used.

As the method for purifying the microparticles, the method described in [0034] to [0064] of JP-T-2019-524824 (the term "JP-T" as used herein means a published Japanese translation of a PCT patent application) may be used, and the contents of the publication are incorporated in the present specification by reference.

### <Concentration of ACE2>

To increase the inhibition rate of spike-ACE2 binding, the concentration (activity) of ACE2 of the composition of the invention is preferably 100 ng/ml or more, more preferably 150 ng/ml or more, particularly preferably 200 ng/ml or more.

Specifically, the concentration of ACE2 of the culture supernatant of dental pulp-derived stem cells or the like is preferably in the range. Alternatively, the concentration of ACE2 of the composition containing microparticles derived from a culture supernatant of dental pulp-derived stem cells or the like is preferably in the range.

It is difficult to prepare a composition having a high concentration of ACE2. Of culture supernatants of mesenchymal stem cells, a culture supernatant of dental pulp-derived stem cells has a very high concentration of ACE2 and is preferably used. Similarly, a composition containing microparticles derived from a culture supernatant of dental pulp-derived stem cells prepared by purification from the culture supernatant of dental pulp-derived stem cells or the like has a very high concentration of ACE2 and is preferably used.

Dental pulp-derived stem cells which are genetically modified to increase the concentration of ACE2 may be used. However, a culture supernatant of dental pulp-derived stem cells, adipose-derived stem cells or umbilical cord-derived stem cells which are not genetically modified also has a sufficiently high concentration of ACE2 and can be used. Of culture supernatants of mesenchymal stem cells, a culture supernatant of dental pulp-derived stem cells has a very high concentration of ACE2, and thus a culture supernatant of dental pulp-derived stem cells which are not genetically modified or a composition containing microparticles derived therefrom can be preferably used. Thus, even when it is required that the cells are not genetically modified for the safety or ethical reason for administering the composition of the invention to a human or an animal, the composition of the invention (especially a culture supernatant of dental pulp-derived stem cells or a composition containing microparticles derived therefrom) can be used.

Moreover, using a culture supernatant of dental pulp-derived stem cells or the like produced by the method described below or using microparticles derived from the culture supernatant, the concentration of ACE2 may be increased.

### <Other Components>

The composition of the invention may contain another component in addition to the microparticles depending on the species of the animal as the subject of the administration or the purpose as long as the effects of the invention are not impaired. Examples of the other component include nutritional components, antibiotics, cytokines, protectants, carriers, excipients, disintegrating agents, buffers, emulsifiers, suspending agents, soothing agents, stabilizers, preservatives, antiseptics and the like.

Examples of the nutritional components include fatty acids, vitamins and the like.

Examples of the antibiotics include penicillin, streptomycin, gentamicin and the like.

The carriers may be materials known as pharmaceutically acceptable carriers.

The composition of the invention may be the microparticles themselves or a pharmaceutical composition further containing a pharmaceutically acceptable carrier or excipient or the like. The purpose of the pharmaceutical composition is to promote administration of the microparticles to the subject animal of the administration.

The pharmaceutically acceptable carrier is preferably a carrier (including a diluent) which does not cause significant irritation in the subject animal of the administration and which does not inhibit the biological activity and the characteristics of the administered composition. Examples of the carrier include propylene glycol, (physiological) saline, an emulsion, a buffer, a medium such as DMEM and RPMI and a low-temperature preservation medium containing a component for removing free radicals.

The composition of the invention preferably does not contain specific substances.

For example, the composition of the invention preferably does not contain dental pulp-derived stem cells or the like.

Moreover, the composition of the invention preferably does not contain MCP-1. In this regard, however, cytokines other than MCP-1 may be contained. Examples of the other cytokines include those described in [0014] to [0020] of JP-A-2018-023343 and the like.

The composition of the invention preferably does not contain Siglec-9. In this regard, however, sialic acid-binding immunoglobulin-like lectins other than Siglec-9 may be contained.

The composition of the invention preferably does not substantially contain serum (fetal bovine serum, human serum, sheep serum or the like). Moreover, the composition of the invention preferably does not substantially contain the conventional serum replacements such as knockout serum replacement (KSR).

The amounts (solid amounts) of the other components in the composition of the invention are each preferably 1 mass% or less, more preferably 0.1 mass% or less, particularly preferably 0.01 mass% or less.

### <Production Method of Composition>

The method for producing the composition of the invention is not particularly restricted.

A culture supernatant of dental pulp-derived stem cells or the like prepared by the above method or a commercially purchased culture supernatant of dental pulp-derived stem cells or the like may be used as the composition of the invention.

Moreover, the composition of the invention may be prepared by preparing a culture supernatant of dental pulp-derived stem cells or the like by the above method and subsequently purifying microparticles from the culture supernatant of dental pulp-derived stem cells or the like. Alternatively, the composition of the invention may be prepared by purifying microparticles from a commercially purchased culture supernatant of dental pulp-derived stem cells or the like. The composition of the invention may also be prepared by receiving a composition containing a culture supernatant of dental pulp-derived stem cells or the like which has been disposed of (or by appropriately purifying such a composition) and purifying microparticles from the composition.

The final form of the composition of the invention is not particularly restricted. For example, the composition of the invention may be in a form in which the microparticles are packed in a container with a solvent or a dispersion medium, a form in which the microparticles are formed into gel with gel and packed in a container, a form in which the microparticles are solidified by freezing and/or drying and formulated or packed in a container or another form. Examples of the container include a tube, a centrifuge tube, a bag or the like which is suitable for cryopreservation. The freezing temperature can be, for example, -20°C to -196°C.

### <Applications of Composition>

The composition of the invention is used for administration to a healthy person or a healthy animal. The healthy person or the healthy animal means an asymptomatic human or animal which has not developed any symptom of infection with a virus that uses ACE2 as a receptor. The healthy person or the healthy animal also includes a human or an animal which is asymptomatically infected with a virus that uses ACE2 as a receptor regardless of whether the human or the animal is negative to infection. Here, the composition of the invention is preferably used for administration to a human or an animal which is negative to infection with a virus that uses ACE2 as a receptor. However, it is not necessary to determine a negative human or animal through preliminary diagnosis, and administration to a human or an animal which is negative and/or asymptomatically infected is preferable in view of efficient prevention.

The composition of the invention can be used as a medicine for preventing infection with a virus that uses ACE2 as a receptor. In particular, the composition of the invention can be used as a medicine for preventing COVID-19. The composition of the invention, however, may be used for an application other than a medicine for preventing COVID-19. In all cases, the composition of the invention is preferably a pharmaceutical composition.

The applications of the composition of the invention are explained below.

The composition of the invention contains sufficient ACE2.

When a virus that uses ACE2 as a receptor (such as some of coronaviruses including SARS-CoV) infects a human or a nonhuman animal, the virus binds to ACE2, which is a virus receptor protein found on the surface of cells, and then causes fusion of the virus outer membrane and the cell membrane. For example, in the case of coronaviruses, the spike protein (S protein) binds to ACE2 in the cell membrane of cells of a human (or a nonhuman animal), then is cleaved by a protease and is activated, and the virus outer membrane and the cell membrane are fused. ACE2 on the surface of microparticles contained in the composition of the invention first binds to the coronavirus, then inhibits or suppresses binding, entry or fusion of the coronavirus into cells having ACE2 and protects against infection with the coronavirus. Accordingly, a component which binds to a virus that uses ACE2 as a receptor can be used as a medicine for preventing infection with such a coronavirus. That is, the composition of the invention preferably inhibits binding of a coronavirus to ACE2 of a human or a nonhuman animal and is more preferably an inhibitor of cell entry or cell fusion of a coronavirus into a cell having ACE2. Because the microparticles function as a decoy for a virus, the composition of the invention is preferably a decoy for a coronavirus.

In the composition of the invention, the inhibition rate of binding between the spike protein of a virus that uses ACE2 as a receptor and ACE2 (spike-ACE2 binding) is 10% or more, preferably 40% or more, more preferably 50% or more, particularly preferably 60% or more.

SARS-CoV-2, which is the virus that causes COVID-19, also binds to cells having ACE2 like conventionally known SARS-CoV. Thus, the composition of the invention is more preferably an inhibitor of cell entry or cell fusion of SARS-CoV-2 into a cell having ACE2. The composition of the invention, which binds to a virus that uses ACE2 as a receptor and inhibits binding of the virus to ACE2 of a human or a nonhuman animal (competes or functions as a decoy), can be used as a medicine for preventing COVID-19. Thus, even when pharmacological test results as a medicine for preventing COVID-19 may not have been shown when the present applicant was filed, it can be said that the composition of the invention can be used (or is possibly used) as a medicine for preventing COVID-19.

Having COVID-19 (being infected with the new coronavirus) means that the living body of a human or a nonhuman animal has cells infected with SARS-CoV-2. The infection can be confirmed especially by a method of detection or viral titration from a respiratory tract sample, a method for detecting a blood-circulating SARS-CoV-2-specific antibody or the like, and a known method using PCR is used at this point.

The prevention of COVID-19 means prevention of infection of the living body of a human or a nonhuman animal with SARS-CoV-2 or at least reduction of the possibility of onset thereof. Through administration of the composition of the invention, the possibility that human or animal cells are infected with SARS-CoV-2 becomes low.

The composition of the invention may be used in combination with a medicine for treating COVID-19. The treatment of COVID-19 means reducing or weakening a symptom associated with viral infection (respiratory syndrome, fever or the like) in the living body of a human or a nonhuman animal or shortening the recovery period of the symptom. The viral infection rate (infectivity titer) of a human or animal body preferably decreases through administration of the composition of the invention, and it is more preferable that the virus disappears completely from the living body.

The composition of the invention can be used as a material of the medical device of the invention described below or a replacement part of a medical device.

Compared to the conventional compositions which can be used as medicines for preventing COVID-19, the composition of the invention has advantages such as easiness of mass production, utilization of culture solutions of stem cells which are otherwise disposed of as industrial waste or the like and decreased cost of the disposal of culture solutions of stem cells. In particular, when the culture supernatant of dental pulp-derived stem cells or the like is a culture supernatant of human dental pulp-derived stem cells, human adipose-derived stem cells or human umbilical cord-derived stem cells, the composition of the invention also has advantages of high safety for example from the immunological point of view and less ethical issues in application to a human. When the culture supernatant of dental pulp-derived stem cells or the like is a culture supernatant of dental pulp-derived stem cells or the like of a patient with any of various diseases (viral infection and the like), the application of the composition of the invention to such a patient will be safer and cause less ethical issues.

The composition of the invention is derived from a culture supernatant of dental pulp-derived stem cells or the like and thus is used also for applications in restorative medicine. In particular, a liquid containing a culture supernatant of dental pulp-derived stem cells or the like is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by the composition using a culture supernatant of dental pulp-derived stem cells or the like. Compared to stem cell transplantation, restoration using the composition of the invention does not easily cause neoplastic transformation or the like and is considered to be safer because cell transplantation is not required. Moreover, a culture supernatant of dental pulp-derived stem cells or the like and the composition of the invention have advantages because those with uniformly standardized quality can be used. Because mass production and an efficient administration method can be selected, use with a low cost is enabled.

### [Binding Inhibitor]

In a first aspect, the binding inhibitor of the invention is a binding inhibitor containing a culture supernatant of dental pulp-derived stem cells, adipose-derived stem cells, umbilical cord-derived stem cells or immortalized stem cells thereof, and the binding inhibitor contains angiotensin-converting enzyme 2 (ACE2), is used for administration to a healthy person or a healthy animal and inhibits binding of a virus that uses ACE2 as a receptor to ACE2 of a human or a nonhuman animal.

In a second aspect, the binding inhibitor of the invention is a binding inhibitor containing microparticles derived from a culture supernatant of dental pulp-derived stem cells, adipose-derived stem cells, umbilical cord-derived stem cells or immortalized stem cells thereof. The microparticles contain ACE2, and the binding inhibitor is used for administration to a healthy person or a healthy animal and inhibits binding of a virus that uses ACE2 as a receptor to ACE2 of a human or a nonhuman animal.

The binding inhibitor of the invention is used for administration to a healthy person or a healthy animal for preventing infection with a virus that uses ACE2 as a receptor rather than treating many symptoms and diseases associated with infection with a virus that uses ACE2 as a receptor such as COVID-19.

Here, a medicine for treating infection with a virus that uses ACE2 as a receptor cannot prevent infection with the virus that uses ACE2 as a receptor.

Accordingly, the binding inhibitor of the invention is clearly distinguished from a medicine for treating infection with a virus that uses ACE2 as a receptor, and the subject group of prevention and/or the subject disease is different in that the binding inhibitor is administered to a healthy person or a healthy animal based on the difference in mechanism of action. This point also applies to the composition of the invention.

The age of the human or the like (the human or the nonhuman animal) as the subject to which the binding inhibitor of the invention is administered is not particularly restricted. The binding inhibitor of the invention may be administered to a young human or the like or administered to an aged human or the like. In the case of viral infection in which aged humans or the like are at a higher risk of severe condition upon infection and in which young humans or the like have mild symptoms or are asymptomatic (for example, COVID-19), the subject group of prevention as the subject of administration is preferably limited to aged humans or the like in view of efficient prevention.

In the case of viral infection in which the group at a high risk of severe condition is identified to some extent (for example, COVID-19), the subject group of prevention as the subject of administration is preferably limited to a group at a high risk of severe condition in view of efficient prevention. The risk factors of severe condition include increased age as mentioned above, humans or animals with underlying diseases, pregnancy, history of smoking and the like. The underlying diseases include chronic obstructive pulmonary disease (COPD), chronic kidney disease, diabetes, hypertension, cardiovascular disease, obesity and the like.

### [Medical Device]

The medical device of the invention contains the composition of the invention.

Examples of the medical device include a mask, a mask filter, a face shield, a spatial virus eliminator and the like.

The mask containing the composition of the invention may be a mask having at least one mask filter having the composition of the invention (a virus-binding filter). The mask filter having the composition of the invention can be produced by adding the composition of the invention to a known filter by a known method such as coating and immersion. The mask filter having the composition of the invention may be freely taken out from the mask, and the mask filter may be replaced freely. The mask containing the composition of the invention contains ACE2 and thus can adsorb a virus such as SARS-CoV-2 and inhibit penetration thereof.

The face shield containing the composition of the invention can be produced by adding the composition of the invention to a known face shield by a known method such as coating and immersion. Alternatively, a face shield may be produced after adding the composition of the invention to a resin sheet (an acrylic sheet or the like) or the like used as the material of the face shield. The face shield containing the composition of the invention contains ACE2 and thus can adsorb a virus such as SARS-CoV-2 and inhibit penetration thereof.

The spatial virus eliminator containing the composition of the invention may be a spatial virus eliminator which can be filled with the composition of the invention and can continuously or intermittently emit the composition. The method for continuously or intermittently emitting the composition of the invention is not particularly restricted. A power such as electricity may be used, or the composition may be released by the heat of a living body, chemical reaction or the like. Alternatively, a spraying device or the like may be driven manually. The shape of the spatial virus eliminator may be a pendant-type shape having a string enabling wearing on the living body, a shape having a fixing component for fixing in a space or a mobile shape such as sprayer. The spatial virus eliminator can be freely filled with the composition of the invention, and the composition of the invention can be replaced freely. The spatial virus eliminator containing the composition of the invention can continuously or intermittently emit a composition or microparticles containing ACE2 and thus can adsorb a virus such as SARS-CoV-2 and inhibit entry of the virus into the living body of a human or the like.

### [Prevention Method of COVID-19]

The method for preventing COVID-19 of the invention includes a step of administering the composition of the invention to a human or a nonhuman animal or a step of installing the medical device of the invention in a human or a nonhuman animal.

The step of administering the composition of the invention to a human or a nonhuman animal is not particularly restricted.

The administration method can be spraying or inhalation to the oral cavity, the nasal cavity or the respiratory tract, infusion, local administration, nasal drops or the like and is preferably little invasive. The local administration method is preferably an injection. Another preferable method is electroporation, in which voltage (electric pulses) is applied to the skin surface to create temporal fine holes in the cell membrane, allowing the active ingredient to penetrate to the dermis layer, which cannot be reached by normal care.

The composition of the invention which has been administered to an animal circulates in the body of the animal and may bind to tissue (cells or stem cells) having ACE2.

The number of administrations and the intervals of administration are not particularly restricted. The number of administrations can be once to twice or more and is preferably once to five times, more preferably once or twice, particularly preferably twice. The intervals of administration are preferably 1 to 24 hours, more preferably within 12 hours. However, the number and the intervals can be appropriately adjusted in accordance with the wishes of the subject of administration.

The timing of administration and the duration of administration are not particularly restricted as long as the administration is at or before the time at which infection with the virus is to be prevented. The composition may be administered continuously every day or administered at appropriate intervals when infection with the virus is to be prevented. The composition may be administered only when the subject of administration wishes, for example while the subject is out or in contact with others, like a mask or a face shield.

When continuous administration to a predetermined person (a close contact, a returnee or a traveler from abroad, a person leaving the country and going abroad or the like) for a certain period is required, the composition may be administered continuously every day during the certain period. When the composition is administered to a medical worker such as a doctor and a nurse who frequently comes into contact with people with viral infection, the composition may be administered only when the subject comes into contact with a person with viral infection (at each shift or the like). In these cases, the composition of the invention is preferably produced in the form of a medical kit or a medical package including daily doses provided for appropriate continuous administration or administration with intervals, like contraceptive drugs. Placebo drugs may be appropriately provided according to the intervals. Alternatively, when the infection is to be prevented with one administration over a long period of time, such as one week or longer, like vaccines, a large dose may be administered at one time.

The animal (species) as the subject to which the composition of the invention is administered is not particularly restricted. The animal as the subject to which the composition of the invention is administered is preferably a mammal, a bird (chicken, quail, duck or the like) or fish (salmon, trout, tuna, bonito or the like). The mammal may be a human or a nonhuman mammal but is particularly preferably a human. The nonhuman mammal is more preferably a cow, a pig, a horse, a goat, a sheep, a monkey, a dog, a cat, a mouse, a rat, a guinea pig or a hamster.

The step of installing the medical device of the invention in a human or a nonhuman animal is not particularly restricted. Depending on the form of the medical device of the invention, the medical device of the invention can be installed in a human or a nonhuman animal. The animal as the subject in which the medical device of the invention is installed is not particularly restricted, and a preferable range thereof is the same as that of the animal as the subject to which the composition of the invention is administered.

### Examples

The characteristics of the invention are explained further specifically referring to Examples and a Comparative Example or a Reference Example below. The materials, the amounts, the proportions, the contents of treatments, the procedures of treatments and the like shown in the Examples below can be appropriately changed as long as such changes do not go beyond the intention of the invention. Accordingly, the scope of the invention should not be construed as being limited by the specific examples shown below.

### [Example 1]

### <Preparation of Culture Supernatant of Dental Pulp-Derived Stem Cells>

A culture supernatant of deciduous dental pulp stem cells was prepared according to the method described in Example 6 of patent No. 6296622 except that DMEM medium was used instead of the DMEM/HamF 12 mixture medium. For the primary culture, fetal bovine serum (FBS) was added to the culture. In the passaging culture, the supernatant was taken from the passaged culture solution which was cultured using the primary culture solution in such a manner that FBS would not be contained, and a culture supernatant of deciduous dental pulp stem cells was thus prepared. Here, DMEM is Dulbecco's modified Eagle's medium, and F12 is Ham's F12 medium.

The obtained culture supernatant of deciduous dental pulp stem cells was used as the composition of Example 1.

### [Example 2]

A culture supernatant of adipose-derived stem cells was prepared in the same manner as in Example 1 except for using adipose-derived stem cells. The obtained culture supernatant of adipose-derived stem cells was used as the composition of Example 2.

### [Evaluation of Inhibition of Spike-ACE2 Binding of Culture Supernatants]

The inhibition of spike-ACE2 binding of the compositions of Examples 1 and 2 was evaluated by the following method using COVID-19 Spike-ACE2 binding assay kit (product No.: CoV-SACE2; manufactured by RayBiotech). In each well, SARS-CoV-2 Spike RBD recombinant protein was immobilized in advance.

### <Preparation of Reagents>

(1) 5× Assay Diluent (Item E2) was diluted 5-fold with distilled water, and a necessary amount of 1× Assay Diluent was thus prepared.
(2) 20× Assay Diluent (Item B) was diluted 5-fold with distilled water, and a necessary amount of 1× Wash Buffer was thus prepared.
(3) To ACE2 Protein (Item F), 50 µl of 1× Assay Diluent obtained in (1) above was added, and ACE2 Protein Concentrate was thus prepared.
(4) To one vial of 1000× Detection antibody (Item C), 100 µl of 1× Assay Diluent obtained in (1) above was added.
(5) The solution obtained in (4) above was diluted 55-fold with 1× Assay Diluent obtained in (1) above, and a necessary amount of ACE2 Detection antibody solution was thus prepared.
(6) HRP-conjugated anti-goat IgG was diluted 1000-fold with 1× Assay Diluent obtained in (1) above, and 1× HRP-conjugated anti-goat IgG solution was thus prepared.

### <Preparation of Test Samples>

ACE2 Protein Concentrate in a volume of 0.7 µl was mixed in 50 µl of the compositions of Examples 1 and 2, and the mixtures were diluted with 1× Assay Diluent to the total volume of 100 µl.

### <Measurement Protocol>

The reagents were brought to room temperature.

The mixtures in the total volume of 100 µl obtained by mixing 0.7 µl of ACE2 Protein Concentrate in 50 µl of the compositions of Examples 1 and 2 and diluting with 1× Assay Diluent were added to the wells of an included microplate. The microplate was covered and incubated with shaking at room temperature for 2.5 hours.

After discarding the solutions in the wells, 300 µl of 1× Wash Buffer was added to the wells to wash the wells four times. ACE2 Detection antibody solution (goat-anti-ACE2 antibody) in a volume of 100 µl was added, and the microplate was incubated with shaking at room temperature for an hour.

After discarding the solutions in the wells, 300 µl of 1× Wash Buffer was added to wash the wells four times. 1× HRP-conjugated anti-goat IgG solution in a volume of 100 µl was added, and the microplate was incubated with shaking at room temperature for an hour.

After discarding the solutions in the wells, 300 µl of 1× Wash Buffer was added to wash the wells four times. TMB (tetramethylbenzidine) One-Step Substrate Reagent in a volume of 100 µl was added, and the microplate was incubated with shaking in a shaded place at room temperature for 10 minutes.

To the wells, 50 µl of Stop solution (a solution to stop the reaction) was added.

The absorbances at 450 nm were measured. The measurement was made while observing the coloration.

The results obtained are shown in Fig. 1.

From Fig. 1, it was found that the compositions of Examples 1 and 2, namely the culture supernatant of dental pulp-derived stem cells and the culture supernatant of adipose-derived stem cells could inhibit spike-ACE2 binding. In particular, it was found that the culture supernatant of dental pulp-derived stem cells (Example 1) had a higher binding inhibition rate than the culture supernatant of adipose-derived stem cells (Example 2).

### [Example 11]

### <Preparation of Exosomes from Culture Supernatant of Dental Pulp-Derived Stem Cells>

To identify the component which would mainly contribute to the inhibition of spike-ACE2 binding, exosomes of the dental pulp-derived stem cells were purified from the culture supernatant of deciduous dental pulp stem cells obtained in Example 1 by the following method.

After filtering the culture supernatant of deciduous dental pulp stem cells (100 mL) with a filter having a pore size of 0.22 micrometers, the solution was centrifuged for 60 minutes at 4°C at 100000× g. The supernatant was decanted, and the exosome-concentrated pellets were re-suspended in phosphate-buffered saline (PBS). The re-suspended sample was centrifuged for 60 minutes at 100000× g. The pellets were again recovered from the bottom of the centrifuge tube as the concentrated sample (about 100 µl). The protein concentration was determined using micro BSA protein assay kit (Pierce, Rockford, IL). The composition containing exosomes (concentrated solution) was stored at -80°C.

The obtained composition containing exosomes was used as the composition of Example 11.

### <Evaluation of Inhibition of Spike-ACE2 Binding>

The inhibition of spike-ACE2 binding was evaluated in the same manner as in Example 1 except that the dental pulp-derived stem cell EVs (the composition of Example 11) in the total protein amount of 2 µg were used instead of 50 µl of the culture supernatant of dental pulp-derived stem cells (the composition of Example 1).

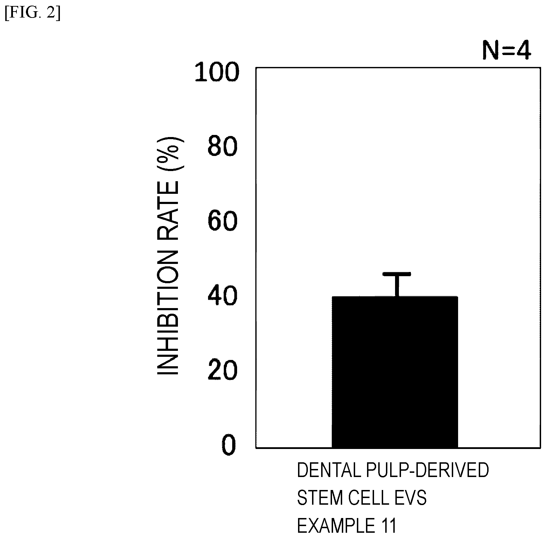

The results obtained are shown in Fig. 2.

From Fig. 2, it was found that the dental pulp-derived stem cell EVs (the composition of Example 11) could inhibit spike-ACE2 binding. The binding inhibition rate of the dental pulp-derived stem cell EVs was 40.8%.

Moreover, as a result of the same evaluation in which the dental pulp-derived stem cell EVs (the composition of Example 11) in terms of the total protein amount were increased from 2 µg, it was found that the binding inhibition rate further increased.

The results in Fig. 1 and Fig. 2 show that the component which mainly contributes to the inhibition of spike-ACE2 binding in the culture supernatants of dental pulp-derived stem cells and the like is exosomes.

### [Example 12]

A composition containing exosomes was prepared from the culture supernatant of adipose-derived stem cells obtained in Example 2 in the same manner as in Example 11. The obtained composition containing exosomes was used as the composition of Example 12.

### [Example 13]

A culture supernatant of umbilical cord-derived stem cells was prepared in the same manner as in Example 1 except for using umbilical cord-derived stem cells. A composition containing exosomes was prepared from the obtained culture supernatant of umbilical cord-derived stem cells in the same manner as in Example 11. The obtained composition containing exosomes was used as the composition of Example 13.

### [Reference Example 1]

A culture supernatant of bone marrow-derived stem cells was prepared in the same manner as in Example 1 except for using bone marrow-derived stem cells. A composition containing exosomes was prepared from the obtained culture supernatant of bone marrow-derived stem cells in the same manner as in Example 11. The obtained composition containing exosomes was used as the composition of Reference Example 1.

### [Evaluation]

### <Characteristics of Exosomes>

The average particle sizes of the microparticles contained in the compositions of Examples 1, 2 and 11 to 13 were 50 to 150 nm.

The compositions of Examples 11 to 13 were exosome solutions having a high concentration of 1.0×10⁹ particles/µL.

Moreover, the components of the obtained compositions of Examples 1, 2 and 11 to 13 were analyzed by a known method. As a result, it was found that the compositions of the Examples did not contain any stem cells such as dental pulp-derived stem cells, did not contain MCP-1 and did not contain Siglec-9. It was thus found that an active ingredient which is different from MCP-1 and Siglec-9, which are active ingredients of a culture supernatant of mesenchymal stem cells, and from analogs thereof is the active ingredient of the compositions of the Examples.

### <Observation of ACE2 Expression>

Using an ACE2 antibody (product No.: 21115-1-AP; manufactured by Cosmo Bio Co., Ltd.) which is a polyclonal antibody, the expression of ACE2 in the compositions of Examples 1, 2 and 11 to 13 or the Reference Example was observed by the following method.

Exosomes of PNT2 cells, which are reported to express ACE2, were prepared, and a composition containing the exosomes was used as PNT2 EVs.

The expression levels of ACE2, namely the amounts of ACE2 protein, in the culture supernatant of dental pulp-derived stem cells (the composition of Example 1), the culture supernatant of adipose-derived stem cells (the composition of Example 2), the dental pulp-derived stem cell EVs (the composition of Example 11), the adipose-derived stem cell EVs (the composition of Example 12), the umbilical cord-derived stem cell EVs (the composition of Example 13), the bone marrow-derived stem cell EVs (the composition of Reference Example 1) and the PNT2 EVs were measured by the ELISA method.

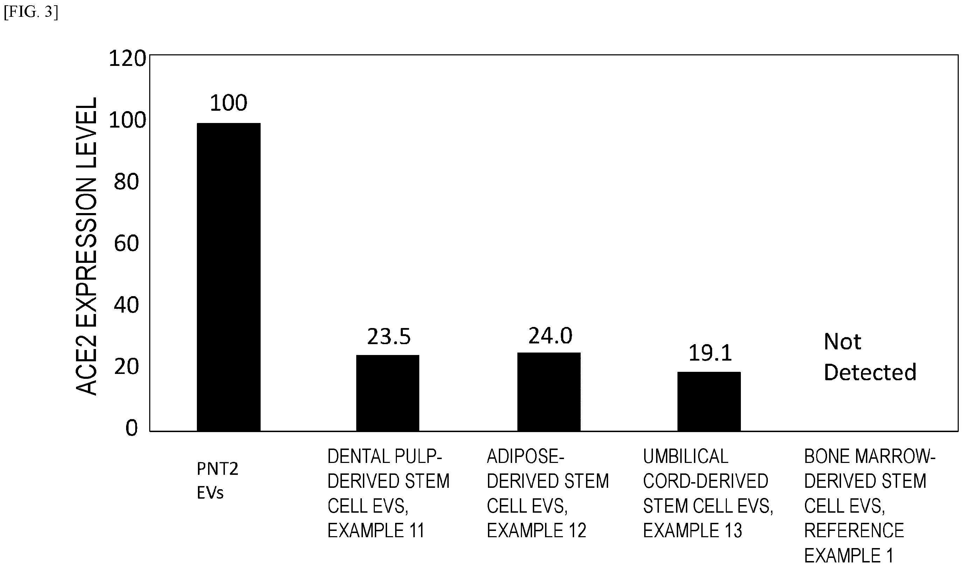

The relative expression levels of ACE2 in the exosomes contained in the compositions of the Examples and the Reference Example were determined (N=1) where the expression level of the PNT2 EVs used as the positive control was regarded as 100. Of the results obtained, the results of Examples 11 to 13 and Reference Example 1 are shown in Fig. 3.

From Fig. 3, it was found that ACE2 was expressed and contained in the exosomes contained in the compositions of the Examples. In particular, it was found that the expression levels of ACE2 in the dental pulp-derived stem cell EVs (Example 11) and the adipose-derived stem cell EVs (Example 12) were higher than that in the umbilical cord-derived stem cell EVs (Example 13). The results showed that the composition of the invention can be used as a medicine for preventing COVID-19. Moreover, binding to a virus that uses ACE2 as a receptor becomes easier as the expression level of ACE2 is higher, and this is more advantageous for the prevention of COVID-19.

On the other hand, the expression of ACE2 was not detected from the bone marrow-derived stem cell EVs (Reference Example 1). From the results, it was unclear whether exosomes derived from a culture supernatant of bone marrow-derived stem cells can be used as a medicine for preventing COVID-19.

### <Measurement of ACE2 Concentrations>

Using SensoLyte (registered trademark) 390 ACE2 Activity Assay Kit, Fluorimetric (manufactured by AnaSpec), the ACE2 concentrations (activities) of the dental pulp-derived stem cell EVs (the composition of Example 11), the adipose-derived stem cell EVs (the composition of Example 12), the umbilical cord-derived stem cell EVs (the composition of Example 13) and the bone marrow-derived stem cell EVs (the composition of Reference Example 1) were measured (100 assays; N=2).

For the measurement, by adjusting the exosome concentrations of the compositions of the Examples and the Reference Example at 1×10⁹ particles/ml and using the same amounts of the compositions in the Examples and the Reference Example, the amounts of the exosomes were made the same. The results obtained are shown in Table 1 below.

Here, the ACE2 concentration is preferably 100 ng/ml or more, more preferably 150 ng/ml or more, particularly preferably 200 ng/ml or more.

**Table 1]**

| | EVs | ACE2 Concentration (ng/ml) |
|---|---|---|
| Example 11 | Dental Pulp-Derived Stem Cell EVs | 225.3 |
| Example 12 | Adipose-Derived Stem Cell EVs | 124.1 |
| Example 13 | Umbilical Cord-Derived Stem Cell EVs | 178.8 |
| Reference Example 1 | Bone Marrow-Derived Stem Cell EVs | 56.6 |

From Table 1 above, it was found that the ACE2 concentrations of the compositions of the Examples were high. In particular, it was found that the ACE2 concentration of the dental pulp-derived stem cell EVs (Example 11) was higher than those of the adipose-derived stem cell EVs (Example 12) and the umbilical cord-derived stem cell EVs (Example 13). The observation of the ACE2 concentration is more accurate than the observation of the expression of ACE2 as a method for observing the easiness of binding to a virus that uses ACE2 as a receptor. Because binding to a virus that uses ACE2 as a receptor is easier as the ACE2 concentration (activity) is higher, it is more effective as a decoy for the virus and is more advantageous for the prevention of COVID-19.

On the other hand, the ACE2 concentration of the bone marrow-derived stem cell EVs (Reference Example 1) was low.

In this regard, the ACE2 concentration of the composition of Example 1 (the culture supernatant of dental pulp-derived stem cells) was equivalent to the ACE2 concentration of the dental pulp-derived stem cell EVs (Example 11). The ACE2 concentration of the composition of Example 2 (the culture supernatant of adipose-derived stem cells) was equivalent to the ACE2 concentration of the adipose-derived stem cell EVs (Example 12).

## Claims

1. A composition containing a culture supernatant of dental pulp-derived stem cells, adipose-derived stem cells, umbilical cord-derived stem cells or immortalized stem cells thereof,
wherein the composition contains ACE2, wherein ACE2 is angiotensin-converting enzyme 2, and
is used for administration to a healthy person or a healthy animal for preventing infection of the healthy person or the healthy animal with a virus that uses ACE2 as a receptor.

2. A composition containing microparticles derived from a culture supernatant of dental pulp-derived stem cells, adipose-derived stem cells, umbilical cord-derived stem cells or immortalized stem cells thereof,
wherein the microparticles contain ACE2, and
the composition is used for administration to a healthy person or a healthy animal for preventing infection of the healthy person or the healthy animal with a virus that uses ACE2 as a receptor.

3. The composition according to claim 2, wherein the microparticles are microparticles derived from the culture supernatant of dental pulp-derived stem cells.

4. The composition according to claim 2 or 3, wherein the microparticles are exosomes.

5. The composition according to any one of claims 2 to 4, wherein the microparticles are microparticles purified from the culture supernatant.

6. The composition according to any one of claims 2 to 5 which contains 0.01×10⁸ particles/ml or more of the microparticles.

7. The composition according to any one of claims 1 to 6 which contains 100 ng/ml or more of ACE2.

8. The composition according to any one of claims 1 to 7, wherein the dental pulp-derived stem cells, the adipose-derived stem cells or the umbilical cord-derived stem cells used for the culture supernatant are not genetically modified.

9. The composition according to any one of claims 1 to 8 which is used for inhibiting binding of the virus that uses ACE2 as a receptor to ACE2 of a human or a nonhuman animal.

10. The composition according to any one of claims 1 to 9, wherein the inhibition rate of binding between the spike protein of the virus that uses ACE2 as a receptor and ACE2 of a human or a nonhuman animal is 40% or more.

11. The composition according to claim 9 or 10 which is used for inhibiting cell entry or cell fusion of SARS-CoV-2 into a cell having ACE2.

12. The composition according to any one of claims 1 to 11 which is a medicine for preventing COVID-19 in the healthy person or the healthy animal.

13. The composition according to any one of claims 1 to 12, wherein the dental pulp-derived stem cells, the adipose-derived stem cells or the umbilical cord-derived stem cells are human dental pulp-derived stem cells, human adipose-derived stem cells or human umbilical cord-derived stem cells.

14. A binding inhibitor containing a culture supernatant of dental pulp-derived stem cells, adipose-derived stem cells, umbilical cord-derived stem cells or immortalized stem cells thereof,
wherein the binding inhibitor contains ACE2, wherein ACE2 is angiotensin-converting enzyme 2,
is used for administration to a healthy person or a healthy animal and
inhibits binding of a virus that uses ACE2 as a receptor to ACE2 of a human or a nonhuman animal.

15. A binding inhibitor containing microparticles derived from a culture supernatant of dental pulp-derived stem cells, adipose-derived stem cells, umbilical cord-derived stem cells or immortalized stem cells thereof,
wherein the microparticles contain ACE2, and
the binding inhibitor is used for administration to a healthy person or a healthy animal and
inhibits binding of a virus that uses ACE2 as a receptor to ACE2 of a human or a nonhuman animal.

16. A medical device containing at least one of the composition according to any one of claims 1 to 13 and the binding inhibitor according to claim 14 or 15.

17. A method for preventing COVID-19 including a step of administering at least one of the composition according to any one of claims 1 to 13 and the binding inhibitor according to claim 14 or 15 to a human or a nonhuman animal or a step of installing the medical device according to claim 16 in a human or a nonhuman animal.
